# EUROPEAN PATENT APPLICATION

(11) **EP 4 023 283 A1**
(43) Date of publication of application: **06.07.2022**
(21) Application number: 20857475.6
(22) Date of filing: 28.08.2020
(51) Int. Cl.: A61N 1/04

(54) **ELECTROCONDUCTIVE RUBBER SHEET AND METHOD FOR PRODUCING ELECTROCONDUCTIVE RUBBER SHEET**

(30) Priority: 30.08.2019 JP 2019158517
(71) Applicant: Ito Co., Ltd., Tokyo 113-0001 (JP); Otsuka Techno Corporation, Naruto-shi, Tokushima 771-0360 (JP)
(72) Inventor: SASAKI, Makoto, Tokyo 113-0001 (JP); HISAMOTO, Takashi, Naruto-shi, Tokushima 771-0360 (JP); MUKAI, Yoichi, Naruto-shi, Tokushima 771-0360 (JP); MASUDA, Tetsuya, Naruto-shi, Tokushima 771-0360 (JP); KAKUYAMA, Hiroki, Naruto-shi, Tokushima 771-0360 (JP)
(74) Representative: Hoffmann Eitle
(86) International application number: PCT/JP2020/032578
(87) International publication number: WO 2021/039956

(57) **Abstract**

An electroconductive rubber sheet includes a sheet-shaped rubber base material having electrical conductivity and an electroconductive sheet embedded in the rubber base material. This electroconductive rubber sheet can be obtained according to a method for producing an electroconductive rubber sheet that includes, for example, a step of placing an electroconductive sheet and a sheet-shaped rubber material, which has electrical conductivity and an opening in a central portion of the sheet-shaped rubber material, on a mold and a step of pressing the electroconductive sheet and the rubber material at a temperature at which the rubber material is softened.

## Description

### Technical Field

The present invention relates to an electroconductive rubber sheet and a method for producing the electroconductive rubber sheet.

### Background Art

For example, a silicone sheet that contains carbon black (electroconductive material) is heretofore known as an electroconductive rubber sheet. The silicone sheet is produced by kneading carbon black into a silicone base material.

On the other hand, an electrode that brings uniform electric distribution over the entirety of a surface of the electrode while maintaining the flexibility of the electrode is disclosed by, for example, Patent Literature 1. The electrode of Patent Literature 1 is composed of a non-electroconductive flexible sheet, an electroconductive pattern, an adhesive made of electroconductive hydrogel, and a lead wire, and the electroconductive pattern and the lead wire are interposed between the flexible sheet and the adhesive.

### Citation List

### Patent Literature

Patent Literature 1: Japanese Translation of International Application (Kohyo) No. 2006-517130

### Summary of Invention

### Technical Problem

It is difficult for an electroconductive rubber sheet (silicone + carbon black) to control electrical conductivity although the production process is simple. For example, it is difficult to uniformize the resistance value (electrical conductivity) of the sheet over its entirety in an in-plane direction.

On the other hand, an electrode having an electroconductive pattern as in Patent Literature 1 might be able to achieve a uniform resistance value (electrical conductivity) over its entirety in an in-plane direction. However, the electrode of Patent Literature 1 is complex in structure, and, in addition, is complex in a process for producing the electrode.

Therefore, an object of the present invention is to provide an electroconductive rubber sheet that has a simple structure, that is capable of reducing variations in the resistance value (electrical conductivity) of the entirety of the sheet, and that is capable of easily producing the sheet, and is to provide a method for producing the electroconductive rubber sheet.

### Solution to Problem

An electroconductive rubber sheet according to an aspect of the present invention includes a sheet-shaped rubber base material having electrical conductivity and an electroconductive sheet embedded in the rubber base material.

Additionally, a method for producing an electroconductive rubber sheet according to an aspect of the present invention includes a step of placing an electroconductive sheet and a sheet-shaped rubber material, which has electrical conductivity and which has an opening formed in a central portion of the rubber material, on a mold, and a step of pressing the electroconductive sheet and the rubber material at a temperature at which the rubber material is softened.

### Advantageous Effects of Invention

According to the electroconductive rubber sheet according to an aspect of the present invention, the electroconductive sheet is embedded in the rubber base material. This makes it possible to lessen variations in the resistance value (electrical conductivity) of the entire rubber sheet by use of the electroconductive sheet even if variations exist in the resistance value (electrical conductivity) of the main body of the rubber base material. Additionally, unlike an aspect in which an electroconductive substance is printed on the rubber base material, the electroconductive sheet never comes into contact with the skin or the finger of the human body when the electroconductive rubber sheet is used. Therefore, it is possible to prevent the electroconductive sheet from being slipped down or being peeled off when the electroconductive rubber sheet is used. As a result, it is possible to maintain the quality of the electroconductive rubber sheet for a long time.

Additionally, according to the electroconductive-rubber-sheet production method according to an aspect of the present invention, it is possible to embed the electroconductive sheet in the rubber material by performing press molding together with the rubber material, and therefore there is no need to add an extra post-processing step. As a result, it is possible to easily produce the electroconductive rubber sheet of the present invention. Additionally, the opening is formed in the central portion of the rubber material, and therefore pressure is not easily applied to the central portion of the rubber material when pressed. As a result, the influence of changes in the local hardness, thickness, etc., of the rubber material becomes small, and it is possible to keep the electroconductive sheet at a desired position. In other words, it is possible to restrain the positional deviation of the electroconductive sheet when pressed.

### Brief Description of Drawings

[FIG. 1] FIG. 1 is a view showing a use situation of an electroconductive rubber sheet according to a preferred embodiment of the present invention.
[FIG. 2] FIG. 2 is a perspective view of the electroconductive rubber sheet according to a preferred embodiment of the present invention.
[FIG. 3] FIG. 3 is a front view of the electroconductive rubber sheet according to a preferred embodiment of the present invention.
[FIG. 4] FIG. 4 is a rear view of the electroconductive rubber sheet according to a preferred embodiment of the present invention.
[FIG. 5] FIG. 5 is a cross-sectional view of the electroconductive rubber sheet according to a preferred embodiment of the present invention, showing a cross section along line V-V of FIG. 3.
[FIG. 6] FIG. 6 is a view showing a part of a production process of the electroconductive rubber sheet according to a preferred embodiment of the present invention.
[FIG. 7] FIG. 7 is a view showing a step subsequent to that of FIG. 6.
[FIG. 8] FIG. 8A and FIG. 8B are views each of which is shown to describe a difference in the adhesive strength of a rubber base material depending on the presence or absence of a mesh.
[FIG. 9] FIG. 9 is a view showing a modification of the electroconductive sheet.
[FIG. 10] FIG. 10 is a view showing a modification of the electroconductive sheet.
[FIG. 11] FIG. 11 is a rear view of an electroconductive rubber sheet according to another preferred embodiment of the present invention.
[FIG. 12] FIG. 12 is a cross-sectional view of the electroconductive rubber sheet of FIG. 11.

### Description of Embodiments

### <Preferred Embodiments of the Present Invention>

First, a preferred embodiment of the present invention will be described in an itemized form.

The electroconductive rubber sheet according to a preferred embodiment of the present invention includes a sheet-shaped rubber base material having electrical conductivity and an electroconductive sheet embedded in the rubber base material.

According to this configuration, the electroconductive sheet is embedded in the rubber base material. This makes it possible to lessen variations in the resistance value (electrical conductivity) of the entire rubber sheet by use of the electroconductive sheet even if variations exist in the resistance value (electrical conductivity) of the main body of the rubber base material. Additionally, unlike an aspect in which an electroconductive substance is printed on the rubber base material, the electroconductive sheet never comes into contact with the skin or the finger of the human body when the electroconductive rubber sheet is used. Therefore, it is possible to prevent the electroconductive sheet from being slipped down or being peeled off when the electroconductive rubber sheet is used. As a result, it is possible to maintain the quality of the electroconductive rubber sheet for a long time.

In the electroconductive rubber sheet according to a preferred embodiment of the present invention, the electroconductive sheet may include an electroconductive mesh. In this case, the electroconductive mesh may be a fabric electroconductive mesh. Although a description is given under the condition that the electroconductive sheet is a fabric electroconductive mesh with respect to the following operational effects, the same operational effects can be fulfilled even if the electroconductive sheet is an electroconductive sheet having a plurality of opening portions instead of the electroconductive mesh.

According to this configuration, the rubber base material enters not only the front and rear surfaces of the electroconductive sheet but also the inside of the lattice of the mesh, and fixes the electroconductive sheet, and adheres closely to the electroconductive sheet. This makes it possible to improve the adhesive strength of the rubber base material with respect to the electroconductive sheet, and makes it possible to prevent the electroconductive sheet from being deviated. Additionally, the electroconductive mesh has flexibility, and therefore it is possible to easily stick the electroconductive rubber sheet while being flexed even when a subject (for example, the skin of the human body) onto which the electroconductive rubber sheet is stuck is in a bent state.

In the electroconductive rubber sheet according to a preferred embodiment of the present invention, the rubber base material may include a terminal that is formed at a peripheral edge portion of the rubber base material and that is used for an electrical connection between the rubber base material and an outside, and the electroconductive sheet may be embedded in the rubber base material such that the electroconductive sheet overlaps the terminal. The electroconductive sheet may be disposed while avoiding the terminal.

According to this configuration, the electroconductive sheet overlaps the terminal, and therefore it is possible to efficiently pass electricity input from the outside to the terminal through the entirety of the rubber base material.

A method for producing an electroconductive rubber sheet according to a preferred embodiment of the present invention includes a step of placing an electroconductive sheet and a sheet-shaped rubber base material, which has electrical conductivity and an opening in a central portion of the sheet-shaped rubber base material, on a mold and a step of pressing the electroconductive sheet and the rubber base material at a temperature at which the rubber base material is softened.

According to this method, it is possible to embed the electroconductive sheet in the rubber material by performing press molding together with the rubber material, and therefore there is no need to add an extra post-processing step. As a result, it is possible to easily produce the electroconductive rubber sheet of the present invention. Additionally, the opening is formed in the central portion of the rubber material, and therefore pressure is not easily applied to the central portion of the rubber material when pressed. As a result, the influence of changes in the local hardness, thickness, etc., of the rubber material becomes small, and it is possible to keep the electroconductive sheet at a desired position. In other words, it is possible to restrain the positional deviation of the electroconductive sheet when pressed.

On the other hand, if the opening is not formed in the central portion of the rubber material, there is a concern that, when the softened rubber material flows into the mold, the electroconductive sheet will also flow together therewith and will overflow the mold, and hence the electroconductive sheet cannot be embedded in a position determined according to a design plan.

In the method for producing an electroconductive rubber sheet according to a preferred embodiment of the present invention, the electroconductive sheet may have an opening. Preferably, a plurality of openings are provided so that the electroconductive sheet is reliably fixed and so that deviation or bend does not occur in a plurality of parts of the inside of the electroconductive sheet in the same way as the electroconductive mesh.

According to this method, the softened rubber material spreads over both surfaces, i.e., the front and rear surfaces of the electroconductive sheet when pressed, and spreads over both surfaces, i.e., the front and rear surfaces of the electroconductive sheet through the opening of the electroconductive sheet or through the lattice of the electroconductive mesh. As a result, it is possible to easily embed the electroconductive sheet in the rubber material.

In the method for producing an electroconductive rubber sheet according to a preferred embodiment of the present invention, the electroconductive sheet may include an electroconductive mesh.

According to this method, the rubber material flows not only into the front and rear surfaces of the electroconductive sheet but also into the lattice of the mesh, and is enabled to be fused together with the electroconductive sheet, and therefore it is possible to restrain the positional deviation of the electroconductive sheet.

### <Detailed Description of Preferred Embodiments of the Present Invention>

Next, a preferred embodiment of the present invention will be described in detail with reference to the accompanying drawings.

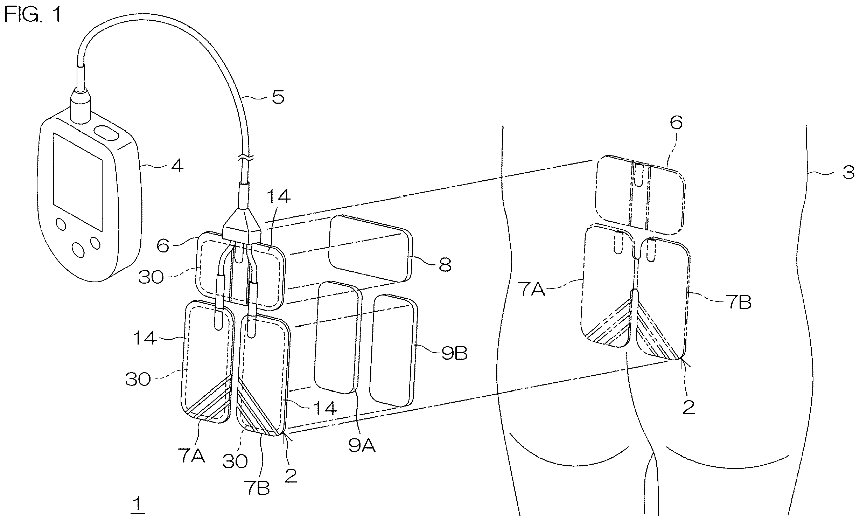

FIG. 1 is a view showing a use situation of an electroconductive rubber sheet 2 according to a preferred embodiment of the present invention.

For example, the electroconductive rubber sheet 2 can be used as an electrode of an electrical stimulation therapeutic device 1, such as a low-frequency therapeutic device. A situation in which the electroconductive rubber sheet 2 is used while being stuck on a back side of the sacrum of a human body 3 is shown as an example of a use situation in FIG. 1.

The electrical stimulation therapeutic device 1 is a device that improves dysuria, such as storage dysfunction (overactive bladder), for example, by giving an electrical stimulus signal from the back side of the sacrum of the human body 3 (person to be treated). The electrical stimulation therapeutic device 1 contributes not only to an improvement in dysuria but also to an improvement in encopresis, such as urge fecal incontinence or passive fecal incontinence, by applying electrical stimulation from the back side of the sacrum.

The electrical stimulation therapeutic device 1 includes the electroconductive rubber sheet 2 serving as an applicator for electrical stimulation treatment, a device body 4, and a wiring cord 5.

The electroconductive rubber sheet 2 includes an indifferent electrode 6 and a pair of stimulating electrodes 7A and 7B. The indifferent electrode 6 and the pair of stimulating electrodes 7A and 7B may be separated from each other as shown in FIG. 1, or may be formed integrally with each other.

Electroconductive adhesion pads 8, 9A, and 9B are stuck onto the indifferent electrode 6 and the pair of stimulating electrodes 7A and 7B, respectively. The indifferent electrode 6 and the pair of stimulating electrodes 7A and 7B are fixed to the skin of the human body 3 by allowing a surface of each of the electroconductive adhesion pads 8, 9A, and 9B to adhere to the skin of the human body 3. In the present preferred embodiment, the electroconductive adhesion pad 8 is stuck onto the indifferent electrode 6, and the electroconductive adhesion pad 9A is stuck onto the stimulating electrode 7A that is one of the pair of stimulating electrodes 7A and 7B, and the electroconductive adhesion pad 9B is stuck onto the other stimulating electrode 7B.

The device body 4 is electrically connected to the indifferent electrode 6 and the pair of stimulating electrodes 7A and 7B by means of the wiring cord 5. A control circuit that includes, for example, a CPU, a memory such as a ROM or RAM, a timer, etc., is built into the device body 4. The person to be treated can output an electrical signal (voltage) for outputting an electrical current of a predetermined frequency to the pair of stimulating electrodes 7A and 7B by operating various operation buttons and a touch panel provided on the device body 4. Hence, electrical stimulation is given to the human body 3 from the pair of stimulating electrodes 7A and 7B.

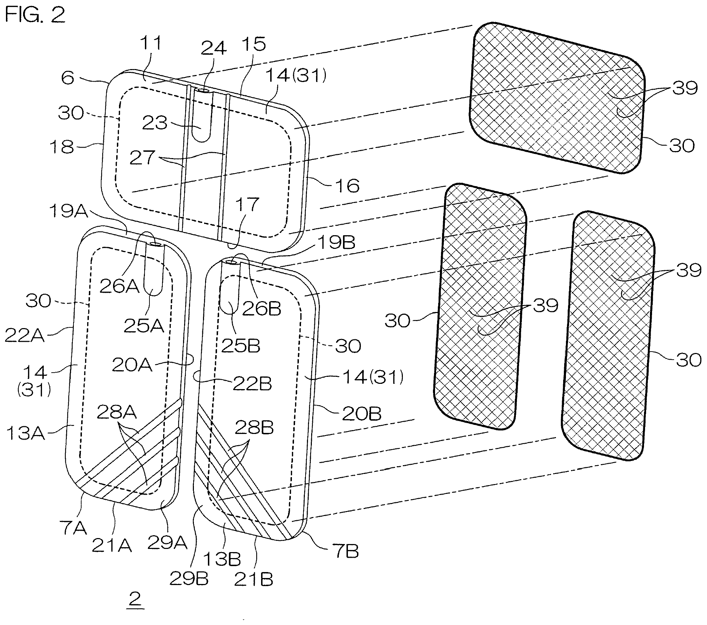

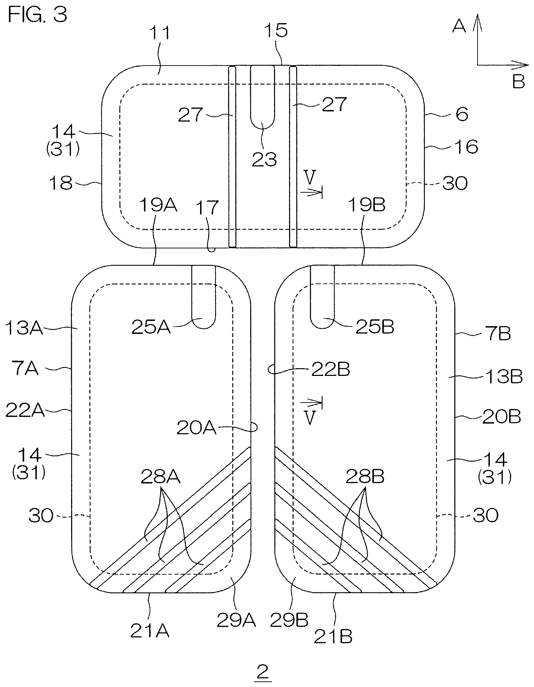

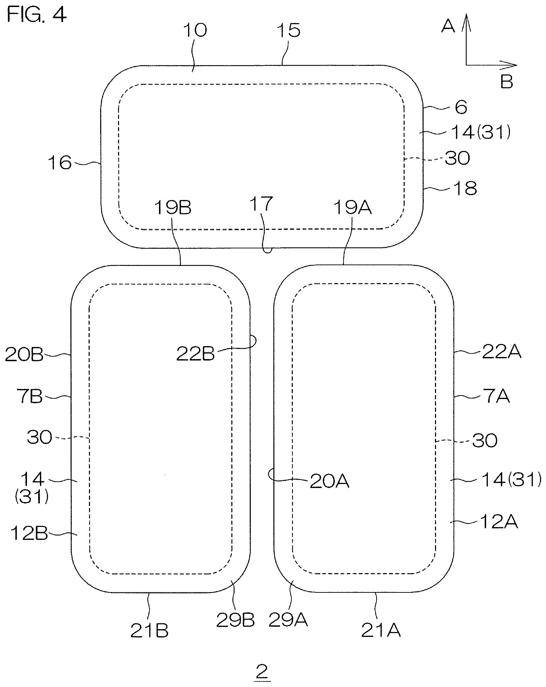

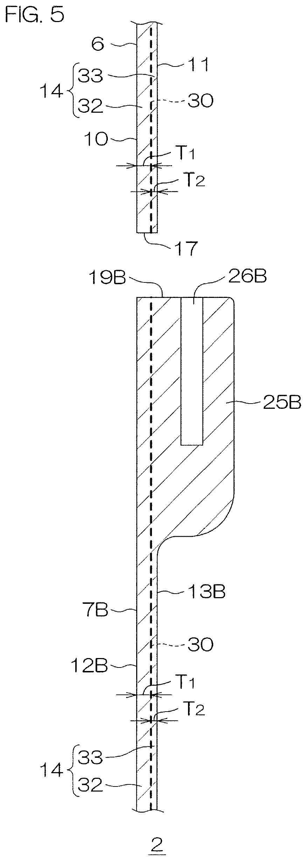

FIG. 2 to FIG. 5 are views each of which is shown to describe a configuration of the electroconductive rubber sheet 2 (the indifferent electrode 6 and the pair of stimulating electrodes 7A and 7B) according to the preferred embodiment of the present invention. FIG. 2 is a perspective view of the electroconductive rubber sheet 2, FIG. 3 is a front view of the electroconductive rubber sheet 2, FIG. 4 is a rear view of the electroconductive rubber sheet 2, and FIG. 5 is a cross-sectional view of the electroconductive rubber sheet 2, showing a cross section along line V-V of FIG. 3. In order to facilitate the understanding of the description, a state in which an electroconductive sheet 30 to be embedded in the rubber base material 14 has not yet been embedded is also shown in FIG. 2.

As described above, the electroconductive rubber sheet 2 includes the indifferent electrode 6 and the pair of stimulating electrodes 7A and 7B.

The indifferent electrode 6 and the pair of stimulating electrodes 7A and 7B have flexibility so that these are adjustably bent when the human body 3 is bent (moved). In the present preferred embodiment, the indifferent electrode 6 and the pair of stimulating electrodes 7A and 7B are each made of a sheet-shaped (plate-shaped) rubber base material 14 that has first surfaces 10, 12A, and 12B that face the skin of the human body 3 and second surfaces 11, 13A, and 13B on the side opposite to the first surfaces 10, 12A, 12B, respectively.

Herein, the "sheet-shaped rubber base material 14" denotes a member most of which is occupied by a region whose thickness is, for example, 0.5 mm to 2.0 mm. Of course, the rubber base material 14 may partly have a structure whose thickness exceeds the thickness falling within the aforementioned range. For example, a first terminal 23, a second terminal 25A, a second terminal 25B, etc., which are described later, can be mentioned as such a structure.

The indifferent electrode 6 is formed in a horizontally-long, substantially quadrangular shape in the present preferred embodiment. The indifferent electrode 6 has a first end portion 15, a second end portion 16, a third end portion 17, and a fourth end portion 18 each of which is an example of a peripheral edge portion of the present invention forming a side of a quadrangular shape.

The first end portion 15 is, for example, an upper end portion of the indifferent electrode 6 when the indifferent electrode 6 is attached to the human body 3, and the first end portion 15 faces the third end portion 17. In other words, the third end portion 17 is a lower end portion of the indifferent electrode 6 when the indifferent electrode 6 is attached to the human body 3. The second end portion 16 and the fourth end portion 18 connect the first end portion 15 and the third end portion 17 together, and face each other.

For example, the indifferent electrode 6 has a length of about 9.5 cm in a lateral direction B along the first and third end portions 15 and 17 and a length of about 5.3 cm in a longitudinal direction A along the second and fourth end portions 16 and 18.

The pair of stimulating electrodes 7A and 7B are each formed in a vertically-long, substantially quadrangular shape in the present preferred embodiment. Each of the stimulation electrodes 7A and 7B has first end portions 19A and 19B, second end portions 20A and 20B, third end portions 21A and 21B, and fourth end portions 22A and 22B each of which is an example of a peripheral edge portion of the present invention forming a side of a quadrangular shape.

The first end portions 19A and 19B are each an upper end portion of each of the stimulation electrodes 7A and 7B, for example, when each of the stimulation electrodes 7A and 7B is attached to the human body 3, and face the third end portions 21A and 21B, respectively. In other words, the third end portions 21A and 21B are each a lower end portion of each of the stimulation electrodes 7A and 7B when each of the stimulation electrodes 7A and 7B is attached to the human body 3. The second end portions 20A and 20B and the fourth end portions 22A and 22B connect the first end portions 19A and 19B and the third end portions 21A and 21B together, and face each other, respectively.

For example, each of the stimulation electrodes 7A and 7B has a length of about 5.3 cm in the lateral direction B along the first end portions 19A and 19B and the third end portions 21A and 21B and a length of about 9.5 cm in the longitudinal direction A along the second end portions 20A and 20B and the fourth end portions 22A and 22B. In other words, the total length in the lateral direction B of the pair of stimulating electrodes 7A and 7B is longer than the length in the lateral direction B of the indifferent electrode 6.

The first terminal 23 is formed integrally with the second surface 11 of the indifferent electrode 6. The first terminal 23 protrudes from the second surface 11 of the indifferent electrode 6. The first terminal 23 has a first entry opening 24 that faces one side (upper side of FIG. 2), and is formed in a cylindrical shape whose opposite side (lower side of FIG. 2) is closed. In the present preferred embodiment, the first entry opening 24 is flush with the first end portion 15 of the indifferent electrode 6.

The second terminals 25A and 25B are formed integrally with the second surfaces 13A and 13B of the pair of stimulating electrodes 7A and 7B, respectively. The second terminals 25A and 25B protrude from the second surfaces 13A and 13B of the pair of stimulating electrodes 7A and 7B. The second terminals 25A and 25B have second entry openings 26A and 26B that follow the same direction as the first entry opening 24, and are each formed in a cylindrical shape whose opposite side (lower side of FIG. 2) is closed. In the present preferred embodiment, the second entry openings 26A and 26B are flush with the first end portions 19A and 19B of the pair of stimulating electrodes 7A and 7B, respectively.

Additionally, a thin portion 27 is formed on the second surface 11 of the indifferent electrode 6. The thin portion 27 is a portion that is comparatively thinly formed in the indifferent electrode 6, and has a thickness of, for example, 0.3 mm to 2.0 mm. The thin portion 27 includes a pair of thin portions 27 that are linear regions (each of which has a width of, for example, about 53 mm) along the second and fourth end portions 16 and 18, respectively.

The pair of thin portions 27 are disposed so as to extend from the first end portion 15 to the third end portion 17 of the indifferent electrode 6 in parallel with each other and so as to interpose the first terminal 23 therebetween. It should be noted that each of the pair of thin portions 27 is away from the first terminal 23 in the directions B along the first and third end portions 15 and 17. The indifferent electrode 6 is formed so as to be easily bent while using the thin portion 27 as a fold because of the formation of the pair of thin portions 27. This makes it possible to excellently stick the indifferent electrode 6 onto the skin of the human body 3 in accordance with the bend of the skin of the human body 3.

Thin portions 28A and 28B are formed on the second surfaces 13A and 13B of the stimulation electrodes 7A and 7B, respectively. The thin portions 28A and 28B are portions that are comparatively thinly formed in the stimulation electrodes 7A and 7B, respectively, and each of the thin portions 28A and 28B has a thickness of, for example, 0.3 mm to 2.0 mm. The thin portion 28A is a linear region (which has a width of, for example, about 53 mm) extending from the second end portion 20A to the third end portion 21A of the stimulation electrode 7A. On the other hand, the thin portion 28B is a linear region (which has a width of, for example, about 53 mm) extending from the fourth end portion 22B to the third end portion 21B of the stimulation electrode 7B.

The thin portions 28A extend from the second end portion 20A to the third end portion 21A of the stimulation electrode 7A in parallel with each other. Additionally, the thin portions 28B extend from the fourth end portion 22B to the third end portion 21B of the stimulation electrode 7B in parallel with each other. In the present preferred embodiment, three thin portions 28A and three thin portions 28B are formed in a stripe manner.

The thin portions 28A and the thin portions 28B are formed in the stimulation electrodes 7A and 7B, respectively, and therefore the stimulation electrodes 7A and 7B are formed so as to be easily bent while using the thin portions 28A and 28B as folds, respectively. This makes it possible to excellently stick each of the stimulation electrodes 7A and 7B onto the skin of the human body 3 in accordance with the bend of the skin of the human body 3. Additionally, the linear thin portions 28A and 28B are formed that connect mutually-adjoining end portions while allowing corner portions 29A and 29B of the stimulation electrodes 7A and 7B to serve as boundaries, respectively, and, in the present preferred embodiment, the linear thin portions 28A and 28B are each formed in a stripe shape sequentially toward an inward region from the corner portions 29A and 29B. Therefore, it is possible to easily unstick each of the stimulation electrodes 7A and 7B from the corner portions 29A and 29B, for example, by picking up the corner portions 29A and 29B with the fingers after treatment.

Every one of the indifferent electrode 6 and the pair of stimulating electrodes 7A and 7B is made of the electroconductive rubber sheet 2 that is composed of the rubber base material 14 and the electroconductive sheet 30 embedded in the rubber base material 14.

The rubber base material 14 forms an external shape of the indifferent electrode 6 and an external shape of each of the pair of stimulating electrodes 7A and 7B. On the other hand, the electroconductive sheet 30 is embedded in the rubber base material 14 by being covered with the rubber base material 14. In FIG. 2 to FIG. 4, a region in which the electroconductive sheet 30 has been embedded is shown by a broken line in the indifferent electrode 6 and in each of the pair of stimulating electrodes 7A and 7B.

The rubber base material 14 is formed by a sheet made of silicone rubber that contains carbon black in the present preferred embodiment. The material of the rubber base material 14 is not limited to silicone rubber that contains carbon black if the rubber base material 14 is rubber having electrical conductivity. For example, silver powder, gold-plated silica or graphite, electroconductive zinc oxide, etc., in addition to carbon black may be employed as a conductor (electroconductive filler) that is mixed with silicone rubber. Additionally, ionic electroconductive silicone rubber may be used as the substance of the rubber base material 14.

The electroconductive sheet 30 is formed by an electroconductive mesh in the present preferred embodiment. For example, a mesh formed by electroconductive fiber, such as silver thread, can be mentioned as the electroconductive mesh.

The electroconductive sheet 30 is embedded in substantially the entirety of the sheet-shaped rubber base material 14. Herein, the "substantially the entirety" denotes that a slight margin (a portion 31 whose entirety in the thickness direction consists of only the rubber base material 14) may be provided between a peripheral edge of the electroconductive sheet 30 and a peripheral edge of the rubber base material 14 (in the present preferred embodiment, end portions 15 to 18, 19A, 19B to 22A, 22B of the indifferent electrode 6 and of the pair of stimulating electrodes 7A and 7B). In the present preferred embodiment, the electroconductive sheet 30 has its entire periphery surrounded by the portion 31 of the rubber base material 14. The size of the margin may be, for example, a size that is set in consideration of a positional deviation of the electroconductive sheet 30 when it is produced.

Therefore, as shown in FIG. 2 and FIG. 3, the electroconductive sheet 30 may overlap the first terminal 23 and the second terminals 25A and 25B disposed at the first end portions 15, 19A, and 19B, respectively, of the indifferent electrode 6 and of the pair of stimulating electrodes 7A and 7B. In other words, in the indifferent electrode 6 and in the pair of stimulating electrodes 7A and 7B, the electroconductive sheet 30 may be embedded in the first terminal 23 and in the second terminals 25A and 25B.

Additionally, in the present preferred embodiment, the electroconductive sheet 30 is disposed disproportionately on the side of the second surfaces 11, 13A, and 13B (surfaces that do not come into contact with the skin of the human body 3) of the rubber base material 14 in the thickness direction of the rubber base material 14. Hence, in a comparison between a thickness T₁ from the electroconductive sheet 30 to the first surfaces 10, 12A, and 12B (surfaces that come into contact with the skin of the human body 3) of the rubber base material 14 and a thickness T₂ from the electroconductive sheet 30 to the second surfaces 11, 13A, and 13B of the rubber base material 14, the thickness T₁ is larger than the thickness T₂.

In other words, the indifferent electrode 6 and the pair of stimulating electrodes 7A and 7B (the electroconductive rubber sheet 2) may have a first portion 32 of the rubber base material 14 having the comparatively large thickness T₁, the electroconductive sheet 30, and a second portion 33 of the rubber base material 14 having the comparatively small thickness T₂ in this order from the side of the first surfaces 10, 12A, and 12B. In still other words, the indifferent electrode 6 and the pair of stimulating electrodes 7A and 7B (the electroconductive rubber sheet 2) may have a three-layer structure of a first rubber layer 32 having the comparatively large thickness T₁, the electroconductive sheet 30, and a second rubber layer 33 having the comparatively small thickness T₂ in this order from the side of the first surfaces 10, 12A, and 12B.

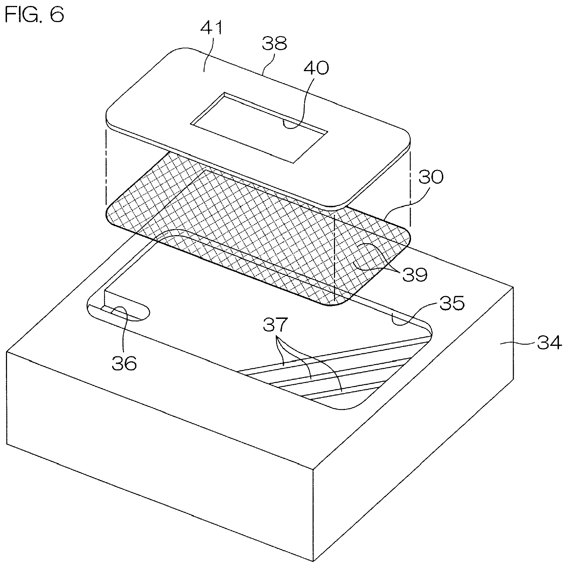

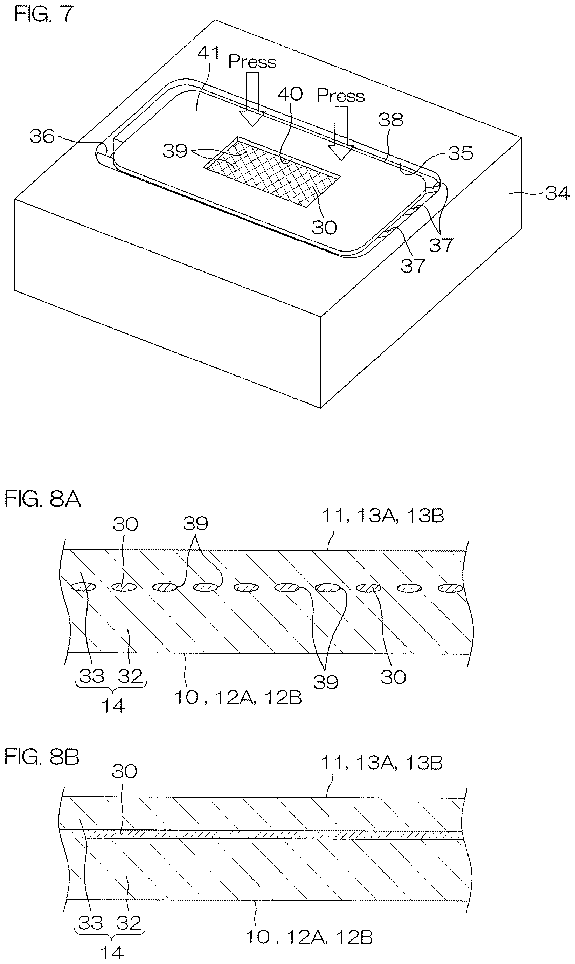

FIG. 6 and FIG. 7 are views each of which shows a part of a production process of the indifferent electrode 6 and the pair of stimulating electrodes 7A and 7B. Herein, production steps of the stimulation electrode 7A are described as an example. The indifferent electrode 6 and the stimulation electrode 7B can be produced according to the production steps of the stimulation electrode 7A, and therefore a description of these production steps of the indifferent electrode 6 and of the stimulation electrode 7B is omitted.

In order to produce the stimulation electrode 7A (the electroconductive rubber sheet 2), a mold 34 for the stimulation electrode 7A is first prepared as shown in FIG. 6. The mold 34 has a concave portion 35 formed in accordance with an external shape of the stimulation electrode 7A. A concave portion 36 for terminals according to a shape of the second terminal 25A and a stripe-shaped convex portion 37 according to a shape of the thin film portion are additionally formed in the concave portion 35 of the mold 34.

On the other hand, a rubber material 38 that is used for a substance for the electroconductive sheet 30 and for the rubber base material 14 is prepared. The electroconductive sheet 30 is formed in a rectangular shape. In the present preferred embodiment, an electroconductive mesh cut in a predetermined shape is prepared. The electroconductive sheet 30 has many openings 39 (lattice-shaped window parts) in its surface.

The rubber material 38 may be a sheet-shaped member formed in a rectangular shape that is substantially equivalent in size to the electroconductive sheet 30. In the present preferred embodiment, the rubber material 38 that is thicker (for example, a thickness of 1.0 mm to 3.0 mm) than the rubber base material 14 is prepared. Additionally, for example, an outer peripheral length of the rubber material 38 may be equal to an outer peripheral length of the electroconductive sheet 30. In other words, in the present preferred embodiment, the rubber material 38 is formed at a smaller size than the size of the concave portion 35 of the mold 34 (i.e., than the size of the stimulation electrode 7A or 7B that is a finished piece) .

An opening 40 is formed in a central portion of the rubber material 38. Therefore, it can be said that the rubber material 38 is formed in an annular shape (in the present preferred embodiment, a quadrangular annular shape). Herein, the "central portion of the rubber material 38" denotes, for example, a fixed region including the center (gravity center) of the rubber material 38. Therefore, the center of the rubber material 38 and the center of the opening 40 are not necessarily required to coincide with each other, and may deviate from each other.

Additionally, no limitations are imposed on the size of the opening 40 of the rubber material 38 if a rubber part 41 that roughly enables the shape of the stimulation electrode 7A to be appropriately molded remains in a region excluding the opening 40. Additionally, it is preferable to create the shape and the number of the opening 40 of the rubber material 38 so that, as described later, pressure cannot be easily applied to the central portion of the rubber material 38 when pressed. A plurality of circular or triangular openings 40 may be formed although, for example, only one quadrangular opening 40 is formed in FIG. 6. Alternatively, a plurality of openings 40 may be provided so as to assume the shape of the electroconductive sheet 30 as a whole. For example, small circular openings 40 may be provided so as to be concentratedly disposed within the range of a rectangular shape corresponding to the shape of the electroconductive sheet 30.

Although the rubber part 41 is formed by the single rubber material 38 in FIG. 6, the present invention is not limited to this, and the rubber 41 and the opening 40 may be formed by a plurality of rubber materials 38. For example, the rubber part 41 and the opening 40 may be formed by arranging two L-shaped rubber materials 38 side by side in a state of facing each other, or the rubber part 41 and the opening 40 may be formed by arranging plate-shaped or rod-shaped slender rubber materials 38 so as to create four sides of a rectangular shape, or the rubber part 41 and the opening 40 may be formed by a plurality of rubber materials 38.

Thereafter, the mold 34 is beforehand heated up to a predetermined temperature (hereinafter, referred to as a "first temperature") that is higher (for example, 170°C to 200°C) than a temperature at which the rubber material 38 is softened, and then the electroconductive sheet 30 and the rubber material 38 are stacked together in this order on an inner surface of the concave portion 35 of the mold 34. The electroconductive sheet 30 is disposed so as to come into contact with the inner surface of the concave portion 35 of the mold 34 and so as to cover a part of the concave portion 36 for terminals. The rubber material 38 is overlapped with the electroconductive sheet 30 so that the outer periphery of the rubber material 38 coincides with the outer periphery of the electroconductive sheet 30.

Thereafter, the electroconductive sheet 30 and the rubber material 38 are pressed and molded by pressing the surface of the rubber material 38 as shown in FIG. 7. Hence, the rubber material 38 that has been softened spreads to the shape of the concave portion 35 of the mold 34, and spreads over both surfaces, i.e., the front and rear surfaces of the electroconductive sheet 30 through the opening 39 of the electroconductive sheet 30. As a result, the electroconductive sheet 30 reaches a state of having been embedded in the rubber material 38 assuming the shape of the rubber base material 14.

Alternatively, the mold 34 may be heated up to a predetermined temperature (hereinafter, referred to as a second temperature, which is, for example, 110°C to 160°C) at which the rubber material 38 is not softened, and then the electroconductive sheet 30 and the rubber material 38 may be stacked together in this order on the inner surface of the concave portion 35 of the mold 34, and the rubber material 38 may be heated by pressing the mold 34 simultaneously with the start of heating the mold 34 up to the first temperature. Thereafter, the mold 34 is cooled, and the rubber base material 14 (rubber material 38) is detached from the mold 34, and, as a result, the stimulation electrode 7A is obtained.

As described above, according to the indifferent electrode 6 and the pair of stimulating electrodes 7A and 7B that are formed by the aforementioned electroconductive rubber sheet 2, the electroconductive sheet 30 is embedded in the rubber base material 14. This makes it possible to lessen variations in the resistance value (electrical conductivity) of the entire electroconductive rubber sheet 2 by use of the electroconductive sheet 30 even if variations exist in the resistance value (electrical conductivity) of the main body of the rubber base material 14. Additionally, unlike an aspect in which an electroconductive substance is printed on the rubber base material 14, the electroconductive sheet 30 never comes into contact with the skin or the finger of the human body 3 when the electroconductive rubber sheet 2 is used. Therefore, it is possible to prevent the electroconductive sheet 30 from being slipped down or being peeled off when the electroconductive rubber sheet 2 is used. As a result, it is possible to maintain the quality of the electroconductive rubber sheet 2 for a long time.

Additionally, in the present preferred embodiment, the electroconductive sheet 30 is an electroconductive mesh. Therefore, the rubber base material 14 enters not only the front and rear surfaces of the electroconductive sheet 30 but also the inside of the opening 39 of the mesh as shown in FIG. 8A, and fixes the electroconductive sheet 30, and adheres closely to the electroconductive sheet 30. This makes it possible to improve the adhesive strength of the rubber base material 14 with respect to the electroconductive sheet 30, and makes it possible to prevent the electroconductive sheet 30 from being deviated. These effects can be achieved even if the electroconductive sheet 30 is not an electroconductive mesh, e.g., even if the electroconductive sheet 30 has a plurality of openings that do not have a mesh structure.

Additionally, the electroconductive mesh has flexibility, and therefore it is possible to easily stick the electroconductive rubber sheet 2 while being flexed even when a subject (for example, the skin of the human body 3, etc.) onto which the electroconductive rubber sheet 2 is stuck is in a bent state.

On the other hand, the electroconductive sheet 30 may be a plate-shaped sheet that has no mesh and no opening as shown in FIG. 8B. It should be noted that the plate-shaped electroconductive sheet 30 comes into contact with the rubber base material 14 only on the front and rear surfaces of the electroconductive sheet 30, and therefore the plate-shaped electroconductive sheet 30 is inferior to the electroconductive sheet 30 having the openings 39 in the adhesive strength with the rubber base material 14. Additionally, the plate-shaped electroconductive sheet 30 is inferior to the electroconductive sheet 30 having the openings 39 in flexibility, and therefore, preferably, the plate-shaped electroconductive sheet 30 is employed as an electrode that is stuck onto a part, which has a comparatively small bend, of the human body 3. When this plate-shaped electroconductive sheet 30 is used, it is recommended to press and mold the electroconductive rubber sheet 2 in a state in which the electroconductive sheet 30 is interposed between a pair of rubber materials 38.

Additionally, in the present preferred embodiment, the thickness T₁ of the first portion 32 of the rubber base material 14 is larger than the thickness T₂ of the second portion 33 as shown in FIG. 5. The second portion 33, i.e., the rubber layer on the side of the second surfaces 11, 13A, and 13B (surfaces not to come into contact with the skin of the human body 3) is a layer formed by allowing a part of the softened rubber material 38 to flow through the openings 39 of the electroconductive sheet 30 when pressed, and it is a principal aim to fix the electroconductive sheet 30. Therefore, it is difficult to control its thickness (i.e., to control its resistance value), and it is not preferable to use this in a state of being in direct contact with the human body 3. On the other hand, the thickness of the rubber layer on the side of the first surfaces 10, 12A, and 12B (surfaces to come into contact with the skin of the human body 3) is somewhat controlled by the thickness of the rubber material 38 prepared at first. Therefore, it is rational to set the first portion 32 having the comparatively large thickness T₁ as the contact side that comes into contact with the skin of the human body 3, and set the second portion 33 having the comparatively small thickness T₂ as the noncontact side that does not come into contact with the skin of the human body 3.

Additionally, according to the present preferred embodiment, the electroconductive sheet 30 overlaps (is embedded in) the first terminal 23 and the second terminals 25A and 25B. On the other hand, the rubber base material 38 has already had electrical conductivity to some extent, and therefore it is not indispensable to allow the electroconductive sheet 30 to overlap a terminal portion, such as the first terminal 23, if it is possible to form a sufficient electric current path between the necessary terminal portion and the electroconductive sheet 30. For example, a configuration may be formed in which the electroconductive sheet 30 is disposed around the terminal portion without being overlapped. Additionally, if a difficulty arises in a pressing operation because of a terminal portion having a complicated shape or a special shape, the electroconductive sheet 30 is disposed while avoiding the terminal portion, which is desirable. Additionally, it is possible to efficiently pass electricity input from the outside into the first and second terminals 23, 25A, and 25B through the entirety of the rubber base material 14, and therefore it is more preferable to allow the electroconductive sheet 30 to overlap the terminal portion as in the present preferred embodiment.

Additionally, according to the production method mentioned above, it is possible to embed the electroconductive sheet 30 by performing press molding together with the rubber material 38, and therefore there is no need to add an extra post-processing step. As a result, it is possible to easily produce the electroconductive rubber sheet 2. Additionally, the opening 40 is formed in the central portion of the rubber material 38, and therefore pressure is not easily applied to the central portion of the rubber material 38 when pressed. As a result, the influence of changes in the local hardness, thickness, etc., of the rubber material 38 becomes small, and it is possible to keep the electroconductive sheet 30 at a desired position when pressed. In other words, it is possible to restrain the positional deviation of the electroconductive sheet 30.

On the other hand, if the opening 40 is not formed in the central portion of the rubber material 38, there is a concern that, when the softened rubber material 38 flows into the mold 34, the electroconductive sheet 30 will also flow together therewith and will overflow the mold 34, and hence the electroconductive sheet 30 cannot be embedded in a position determined according to a design plan.

Additionally, the electroconductive sheet 30 is an electroconductive mesh, and therefore the softened rubber material 38 spreads over both surfaces, i.e., the front and rear surfaces of the electroconductive sheet 30 when pressed, and spreads over both surfaces, i.e., the front and rear surfaces of the electroconductive sheet 30 through the opening 39 of the electroconductive sheet 30. As a result, it is possible to easily embed the electroconductive sheet 30 in the rubber material 38. Particularly, the electroconductive sheet 30 is an electroconductive mesh, and therefore the rubber material 38 flows not only into the front and rear surfaces of the electroconductive sheet 30 but also into the opening 39 of the mesh, and is enabled to be fused together with the electroconductive sheet 30, and therefore it is possible to restrain the positional deviation of the electroconductive sheet 30.

Although the preferred embodiment of the present invention has been described as above, the present invention can be embodied in other modes.

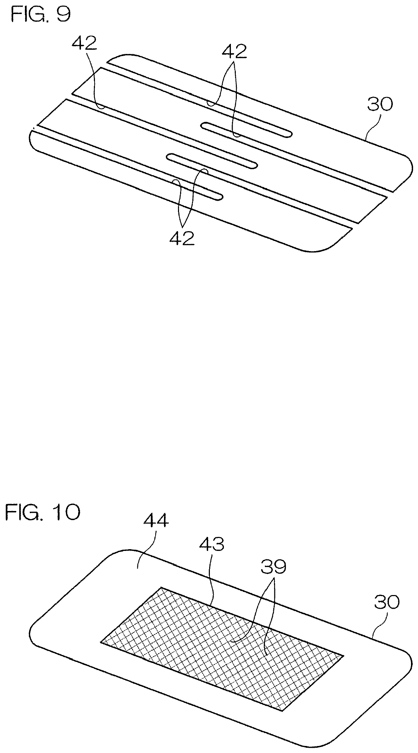

For example, the electroconductive sheet 30 may have cuts 42 or slits 42 formed from the end portion of the electroconductive sheet 30 as shown in FIG. 9 instead of the openings 39 mentioned above. In this case, it is recommended to form cuts 42 or slits 42 so as to be parallel with a bending direction or a curving direction when the electroconductive sheet 30 is used as an electrode. Alternatively, cuts 42 or slits 42 may be arranged in, for example, a radial manner so as to extend from the inside to the end portion of the electroconductive sheet 30 and so as to be nonparallel with each other.

Additionally, a part of the electroconductive sheet 30 may be a mesh as shown in FIG. 10 although the entire surface of the electroconductive sheet 30 may be a mesh as described above. For example, the central portion of the electroconductive sheet 30 may have a mesh portion 43, and a sheet portion 44 may be formed so as to surround the mesh portion 43.

For example, as has been described in the preferred embodiment, the indifferent electrode 6 and the stimulation electrodes 7A and 7B of the electrical stimulation therapeutic device 1 are mentioned as an example of the purpose of use of the electroconductive rubber sheet 2, and yet the electroconductive rubber sheet 2 of the present invention can be employed as, for example, an electrode or the like of an EMS (Electrical Muscle Stimulation) device for physical exercise or for beauty.

Additionally, as has been described in the preferred embodiment, the electroconductive rubber sheet 2 has the first portion 32 of the rubber base material 14 having the thickness T₁, the electroconductive sheet 30, and the second portion 33 of the rubber base material 14 having the thickness T₂ from the side of the first surfaces 10, 12A, and 12B in this order, and the thickness relation therebetween is T₁ > T₂, and yet the thickness relation therebetween may be, for example, T₁ = T₂, or may be T₁ < T₂.

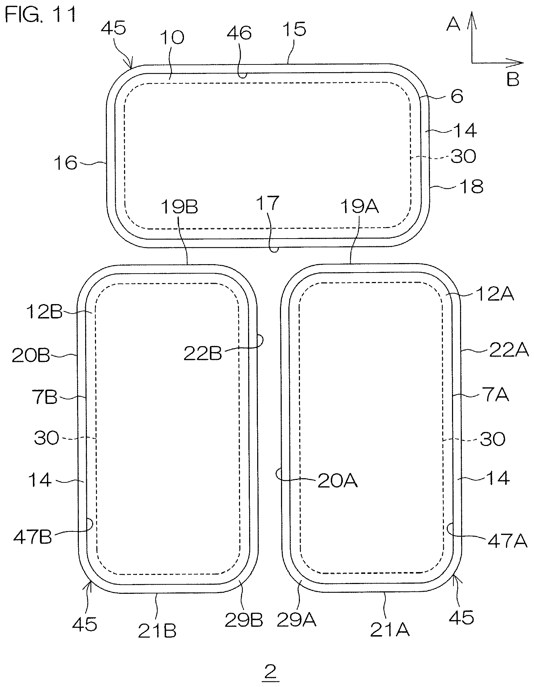

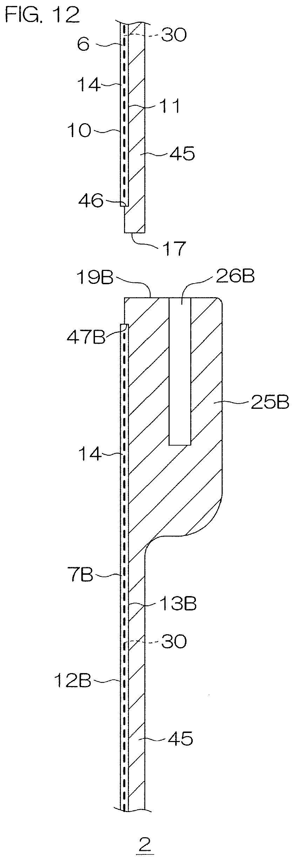

Additionally, as has been described in the preferred embodiment, the external shape of the applicator of the electrical stimulation therapeutic device 1 is formed by the indifferent electrode 6 and the pair of stimulating electrodes 7A and 7B in which the electroconductive sheet 30 has been embedded, and yet its external shape may be formed by a sheet body 45 made of a rubber base material, such as silicone resin, as shown in FIG. 11 and FIG. 12. In this case, the indifferent electrode 6 and the pair of stimulating electrodes 7A and 7B may be each formed in a substantially quadrangular shape. The indifferent electrode 6 and the pair of stimulating electrodes 7A and 7B may be each disposed at concave portions 46, 47A, and 47B, respectively, which are formed in the sheet body 45. The concave portions 46, 47A, and 47B may be identical in shape with the indifferent electrode 6 and the pair of stimulating electrodes 7A and 7B, respectively.

In order to produce the thus formed electroconductive rubber sheet 2 (applicator), for example, the indifferent electrode 6 and the pair of stimulating electrodes 7A and 7B each of which has a substantially quadrangular shape are first formed according to the steps of FIG. 6 and FIG. 7. Thereafter, the indifferent electrode 6 and the pair of stimulating electrodes 7A and 7B are inserted into the mold as insert members, respectively, and the inside of the mold is filled with, for example, silicone resin or the like that is a material for the sheet body 45. Hence, an applicator, which is an insert-molded article, can be obtained.

It should be noted that the method for producing the applicator is not limited to the insert molding described above, and may employ, for example, a method in which the indifferent electrode 6, the pair of stimulating electrodes 7A and 7B, and the sheet body 45 are produced as mutually-different molded articles, and then the indifferent electrode 6 and the pair of stimulating electrodes 7A and 7B are fitted into the concave portions 46, 47A, and 47B, respectively, of the sheet body 45.

This application corresponds to Japanese Patent Application No. 2019-158517 filed in the Japan Patent Office on August 30, 2019, the entire disclosure of which is incorporated herein by reference.

### Reference Signs List

2 electroconductive rubber sheet
6 indifferent electrode
7A, 7B stimulating electrode
14 rubber base material
15 (indifferent electrode) first end portion
19A, 19B (stimulating electrode) first end portion
23 first terminal
25A, 25B second terminal
30 electroconductive sheet
34 mold
38 rubber material
39 opening
40 opening

## Claims

1. An electroconductive rubber sheet comprising:
a sheet-shaped rubber base material having electrical conductivity; and
an electroconductive sheet embedded in the rubber base material.

2. The electroconductive rubber sheet according to claim 1, wherein the electroconductive sheet includes an electroconductive mesh.

3. The electroconductive rubber sheet according to claim 1 or claim 2, wherein the rubber base material includes a terminal that is formed at a peripheral edge portion of the rubber base material and that is used for an electrical connection between the rubber base material and an outside, and
the electroconductive sheet is embedded in the rubber base material such that the electroconductive sheet overlaps the terminal.

4. A method for producing an electroconductive rubber sheet, the method comprising:
a step of placing an electroconductive sheet and a sheet-shaped rubber material on a mold, the sheet-shaped rubber material having electrical conductivity and an opening in a central portion of the sheet-shaped rubber material; and
a step of pressing the electroconductive sheet and the rubber material at a temperature at which the rubber material is softened.

5. The method for producing an electroconductive rubber sheet according to claim 4, wherein the electroconductive sheet has an opening.

6. The method for producing an electroconductive rubber sheet according to claim 5, wherein the electroconductive sheet includes an electroconductive mesh.
